# EUROPEAN PATENT APPLICATION

(11) **EP 4 629 249 A1**
(43) Date of publication of application: **08.10.2025**
(21) Application number: 24168087.5
(22) Date of filing: 02.04.2024
(51) Int. Cl.: G16B 40/10

(54) **PROCESSING AND ANALYSIS OF PCR DATA**

(71) Applicant: Deutsches Krebsforschungszentrum Stiftung des öffentlichen Rechts, 69120 Heidelberg (DE)
(72) Inventor: HÖFLER, Daniela, 69120 Heidelberg (DE); JESKE, Rima, 69120 Heidelberg (DE); ROSING, Fabian, 69120 Heidelberg (DE)
(74) Representative: Vossius & Partner Patentanwälte Rechtsanwälte mbB

(57) **Abstract**

A method for error correction in multichannel raw fluorescence data obtained by digital polymerase chain reaction, dPCR, is provided, the method including the steps of identifying partitions providing aberrant fluorescence readings in a first fluorescence channel, comparing the fluorescence readings of at least some of the partitions providing aberrant fluorescence reading in the first fluorescence channel, with the fluorescence reading of the same partitions in a second fluorescence channel, and adjusting the fluorescence readings of the partitions providing aberrant fluorescence readings in the first fluorescence channel based on the result of the comparison. According to an embodiment, the fluorescence intensity in the partition providing aberrant fluorescence reading in the first fluorescence channel is preferably compared with the fluorescence intensity of the same partition in more than one second fluorescence channel.

## Description

The present disclosure generally relates to the analysis of polymerase chain reaction, PCR, data, in particular a method for processing and analyzing raw data when performing digital PCR.

A digital PCR (dPCR) system allows for the simultaneous quantification of several DNA targets. For example, the QIAcuity system allows the simultaneous quantification of up to five different DNA targets. Information about this specific system can be found at https://www.qiagen.com/us/products/instruments-and-automation/pcr-instruments/qiacuity-digital-pcr-system. This is an advantage compared to other digital PCR systems, which often use only two fluorescence channels.

A dPCR system uses nanoplates, such as nanoplates with 24 or 96 wells, each of which holds one sample per well. In each well, the entire reaction volume is divided into several thousand partitions. In each partition, one or several, in particular 2, 3, 4, 5, or more, different DNA targets are amplified and subsequently detected using fluorescence. Highly precise and sensitive quantification is possible by measuring the fluorescence generated in the individual partitions. For this purpose, a threshold value must be defined, on the basis of which each partition is classified as positive or negative, depending on whether the fluorescence intensity exceeds the threshold value or not. The number of molecules of the DNA target in the sample can be calculated from the number of positive partitions.

The dPCR system can simultaneously quantify several different DNA targets using several different fluorescent dyes. However, fluorescence signals may be detected in several different channels that cannot be clearly assigned to one fluorescent dye due to overlapping emission spectra. The fluorescence signals of the different channels therefore influence each other, resulting in so-called cross-talk. In particular, by way of an example, in a system configured to simultaneously quantify five different DNA targets, five different fluorescent dyes will be used, which may influence each other resulting in cross-talk.

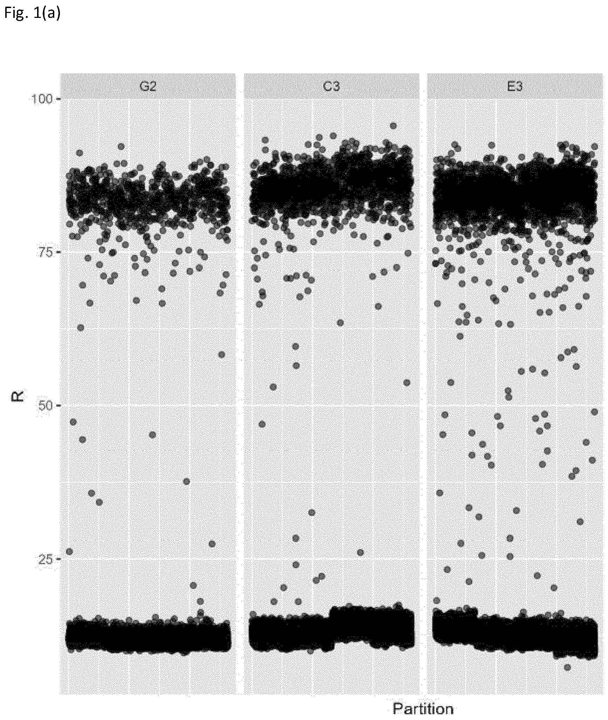

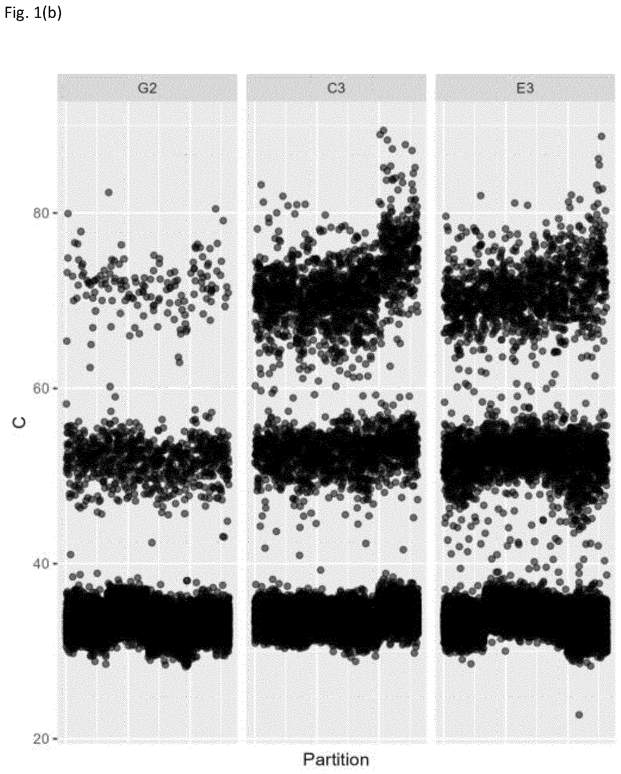

Fig. 1 shows dPCR fluorescence values of two channels, namely (a) channel R without cross-talk and (b) channel C with an additional group of partitions by cross-talk. The y-axis shows the fluorescence intensity in the respective channel, the x-axis shows the position of the partition. While in dPCR without cross-talk each partition is either clearly positive (high fluorescence intensity) or negative (low fluorescence intensity) for a target (Figure 1A), the data becomes much more difficult to interpret when cross-talk occurs (Figure 1B). This is because more than two groups of partitions occur in channels with cross-talk. This can lead to a massive loss in the precision of DNA quantification, especially to false positive results, as it is difficult to set a cut-off value that divides the partitions into positive and negative partitions. With very strong cross-talk, even the results of an entire experiment may not be interpretable.

The presence of many copies of several DNA targets in a sample can cause competition effects, as several DNA targets are distributed in the same partition and amplified there.

Since parallel reactions in a partition compete for resources (dNTPs, polymerase, etc.), this can lead to a weaker fluorescence intensity compared to the fluorescence intensity in partitions with only one DNA target. As a result, one or more additional groups of partitions are present that have intermediate fluorescence intensity. This makes precise quantification more difficult, as this group of partitions may be incorrectly evaluated as negative.

Due to the various interfering signals in the data, it is difficult to set a limit value that can be applied to all wells in one experiment. Although the manufacturer's software allows a separate limit value to be defined for each well, this could lead to the limit values not being selected objectively and the data being distorted. This is particularly the case if the data is ambiguous.

In summary, it can be said that the large number of channels in a dPCR system is advantageous, but it also brings with it problems that severely impair data quality.

US 2021/381980 A1 describes a method for analyzing dPCR data, a fluoresence reader reading up to 7 different fluorescence dyes/channels. Cross-talk correction algorithm via a standard calibration procedure by empirically determining calibration constants is implemented.

WO 2018/151843 A2 describes spectral error correction for sample analysis instruments. Cross-talk matrices containing values corresponding to the relationships between intensity measurements of each of two-dye combinations are obtained from the existing dye data.

WO 2023/043851 A1 describes dPCR wherein spectrum compensation values are generated during a PCR apparatus calibration run.

It is therefore an object of the present disclosure to solve the above problems of multi-channel dPCR systems that use multiple fluorescence channels without requiring a separate calibration procedure. In particular, the technical problems may be compensated by subsequent processing of the raw-data output of the dPCR system to correct for background noise and artifacts, to correct for cross-talk between the channels, and to correct for competitive effects between the parallel reactions.

The basic idea of the present disclosure is the automatic identification and classification of local maxima in the histogram of the fluorescence intensities of the individual partitions, which represent groups of partitions separated from each other by local minima.

The present invention is defined by the independent claims; the dependent claims describe embodiments of the present invention.

The method for error correction in multichannel raw fluorescence data obtained by digital polymerase chain reaction, dPCR, includes the steps of identifying partitions providing aberrant fluorescence readings in a first fluorescence channel, comparing the fluorescence readings of at least some of the partitions providing aberrant fluorescence reading in the first fluorescence channel, with the fluorescence reading of the same partitions in a second fluorescence channel, and adjusting the fluorescence readings of the partitions providing aberrant fluorescence readings in the first fluorescence channel based on the result of the comparison for compensating the error in the raw fluorescence data. According to an embodiment, the fluorescence intensity in the partition providing aberrant fluorescence reading in the first fluorescence channel is preferably compared with the fluorescence intensity of the same partition in more than one second fluorescence channel.

The method may further comprise identifying partitions providing positive and negative fluorescence readings in the first fluorescence channel, wherein partitions providing aberrant fluorescence readings are distinguished from the partitions providing positive and negative fluorescence readings by determining local minimums in a histogram of fluorescence intensities of the partitions registered in the first fluorescence channel. For distinguishing partitions providing aberrant fluorescence readings from the partitions providing positive and negative fluorescence readings, various approaches may be used, such as mixture modeling of multiple normal distributions (see, for example, Benaglia T, Chauveau D, Hunter DR, Young DS. mixtools: An R Package for Analyzing Mixture Models. Journal of Statistical Software. 2009;32(6):1 - 29), or identifying local extrema of a density function fit to the data (see, for example, Luukko PJJ, Helske J, Räsänen E. Introducing libeemd: a program package for performing the ensemble empirical mode decomposition. Computational Statistics. 2016;31(2):545-57). Both of these methods serve to define the distinct groups of partitions, which then need to be classified as a basis for further processing.

Further, in the step of comparing the fluorescence readings, fluorescence intensity in the partitions providing aberrant fluorescence reading in the first fluorescence channel may be compared with fluorescence intensity in the same partitions in the second fluorescence channel, for determining an error type of the aberrant fluorescent reading. In particular, the group of negative partitions may be marked by low fluorescence readings in all channels, while the single positive group in the first channel usually only has high fluorescence readings in the first channel. Usually, these two groups will be the largest in the dataset. The fluorescence intensities of these two groups are highly dependent on the efficiency of the underlying dPCR assay, the choice of fluorescent dye and quencher and the fluorescence detection settings, so that for each experiment these groups need to be identified based on relative fluorescence intensities in all channels and group size. Groups of partitions that deviate from the expected positive and negative fluorescence readings are said to show aberrant fluorescence intensities that could be caused by one of two common kinds of error: cross-talk due to spectral overlap of fluorescent dyes, or competition between two reactions of a multiplex dPCR.

A crosstalk error may be determined as type of error, if a substantial number of the partitions providing aberrant fluorescence reading in the first fluorescence channel provides a positive fluorescence reading in the second fluorescence channel. Usually, this presents in the data as partitions that have an intermediate fluorescence reading in the first channel that is between that of the positive and negative groups, but belong to the group of positive partitions in a second channel. In this case, all partitions which are only positive in the second channel will show increased fluorescence intensities in the first channel. This is usually a linear relationship between the fluorescence readings of the two channels in these partitions.

In particular, a crosstalk error may be determined as type of error, if no or essentially no partitions provide both a positive fluorescence reading in the second fluorescence channel and negative fluorescence reading in the first fluorescence channel. In this case, "essentially no" preferably means less than 5, 3, 2, 1, 0.5, or most preferably 0.1% of the number of partitions showing a positive fluorescence reading in the second fluorescence channel.

Furthermore, a competitive reaction error may be determined as type of error, if a substantial number of the partitions providing aberrant fluorescence reading in the first fluorescence channel gives aberrant fluorescence intensity in both the first channel and at least one other (e.g. the second) channel, and/or if a substantial number of the partitions providing aberrant fluorescence reading in the first fluorescence channel gives a positive fluorescent reading in at least the second channel, provided that also there is a substantial number of partitions providing both a positive fluorescence reading in the second fluorescence channel and negative fluorescence reading in the first fluorescence channel. In particular, a competitive reaction may be determined if partitions are present that have intermediate or high fluorescence readings in two channels at once that clearly differentiate them from both the negative partitions and those two groups of partitions positive in only one of the two channels (single positives). These partitions with aberrant fluorescence represent the group that is positive in both channels, despite the potentially lower fluorescence intensity compared to the single positive groups. Competition effects can also be differentiated from crosstalk effects by unaffected fluorescence readings in the single positive groups.

For compensating the error, a linear regression method may be used, such as, for example, the one described in Thulin M. Modern Statistics with R: Eos Chasma Press; 2021, chapter 3.7 Fitting a linear regression model, by estimating the strength of the error based on fluorescence intensity data from partitions which are affected by the errors and from partitions which are unaffected by the errors. In a preferred embodiment, in order to correct crosstalk, a linear model may be fit to the fluorescence data from partitions negative in both the first and the second channel, and those which are positive in the second channel but have aberrant fluorescence in the first channel. This model can be used to estimate the effect of crosstalk on all partitions in the dataset. In a more preferred embodiment, to ensure that the relevant data are available, a control sample may be included that will yield partitions which are positive for all channels. The correction of competition effects may be achieved similarly, but by fitting the model to the data from partitions that are positive in the second channel but negative in the first, and those which have aberrant fluorescence intensity in both channels. An alternative result of the analysis could also be a classification of the different groups of partitions based on the type of error, which would already make it possible to identify the positive of negative status of partitions with aberrant fluorescence intensities. Data from dPCR devices may be analyzed by the same method if they are transferred into a matrix where each line represents a partition and each column contains only either information about the sample ID and partition ID or fluorescence data. In principle, this method could be used for any set of data with multiple numerical variables that show unintended influence of each other in a manner that is comparable to the behavior of the fluorescence intensities in dPCR.

The fluorescence intensity in the partition providing aberrant fluorescence reading in the first fluorescence channel may also be compared with fluorescence intensity of the same partition in more than one second fluorescence channel, preferably with fluorescence intensities of the same partition in all the available fluorescence channels. According to a preferred embodiment, the method of the present disclosure may be repeated for at least one other fluorescence channel, most preferably for all the available fluorescence channels. "Available fluorescence channels" here are to be understood as all fluorescence channels that are used in a given dPCR experiment for registering fluorescence of different fluorescent dyes and/or detecting different DNA targets.

The present disclosure further provides a machine-readable medium storing instructions that, when executed by a computer, cause it to perform the method according to present disclosure. Moreover, the disclosure encompasses a digital polymerase chain reaction, dPCR, device that is configured to carry out the method of the present disclosure.

In the following, the present disclosure is described in more detail with reference to the figures wherein
- Fig. 1: shows dPCR fluorescence values of two channels, wherein (a) shows channel R without cross-talk, (b) shows channel C with an additional group of partitions by cross-talk;
- Fig. 2: shows a histogram of the signal intensities output in channel C;
- Fig. 3: shows effects of the inventive method in channel O; and
- Fig. 4: shows the effect of cross-talk correction in channel C.

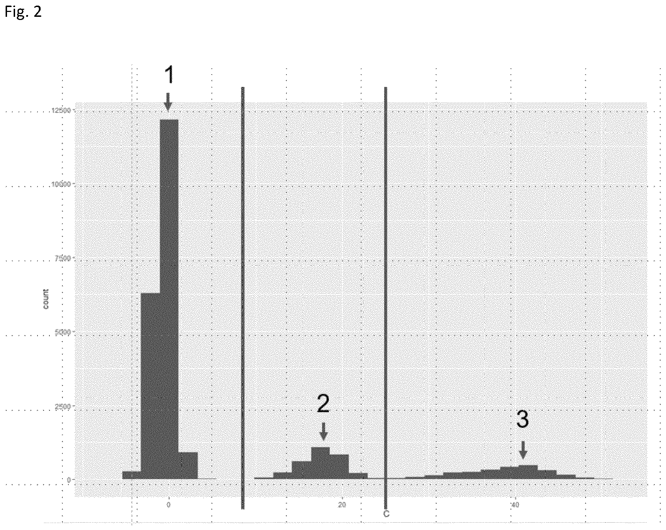

A basis of the method of present disclosure can be seen in the automatic identification and classification of local maxima in the histogram of the fluorescence intensities of the individual partitions, which represents groups of partitions separated from each other by local minima, as shown in Fig. 2. By way of an example, the histogram of Fig. 2 shows the signal intensities output in channel C. The numbers indicated in Fig. 2 mark three local maxima, i.e. peaks 1, 2, and 3, which can be distinguished from each other, the vertical lines mark the minima between the local maxima.

Ideally, two groups of partitions are to be expected, as in Figure 1(a), which represent the positive and negative partitions and can be clearly distinguished from each other. As can be seen in Figure 2, another local maximum (peak 2) is observed in channel C, which makes it difficult to interpret and classify the partitions as positive or negative. If the median fluorescence intensities of the partitions - assigned to the respective groups - are calculated in the other fluorescence channels, it can be seen that all partitions that form peak 2 in channel C have a very high value in channel R, as indicated in the following Table 1:

**Table 1: Median fluorescence intensities of the partitions assigned to the three peaks in two different channels**

| **EAK** | **CHANNEL C** | **CHANNEL R** |
|---|---|---|
| **1** | 0.6 | 0.0 |
| **2** | 17.6 | 63.9 |
| **3** | 38.6 | 11.8 |

This suggests that this local maximum was caused by the cross-talk from channel R to channel C. The strength of this effect cam be determined by a simple linear regression model from the fluorescence values of the double negative (peak 1) and the single positive (peak 2) partitions in channel R. This information can be used to subtract the proportion of cross-talk in the fluorescence intensity for each partition.

The correction of the competition effects follows the same principle, except that this is based on the comparison of the fluorescence values of the double-positive and single-positive partitions.

This procedure facilitates the setting of a threshold value for a clear classification of the positive and negative partitions and reduces the probability of misclassification, as a threshold value can now be set that is applicable to all wells.

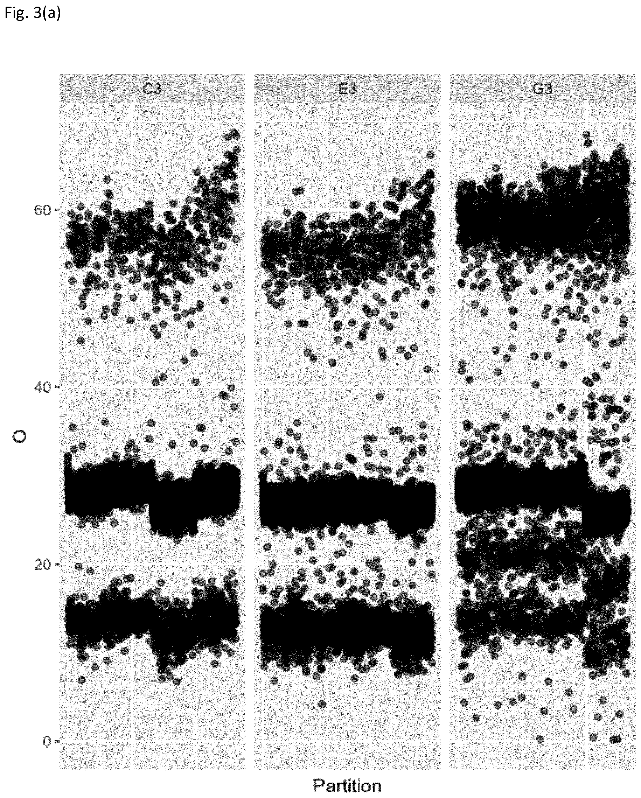

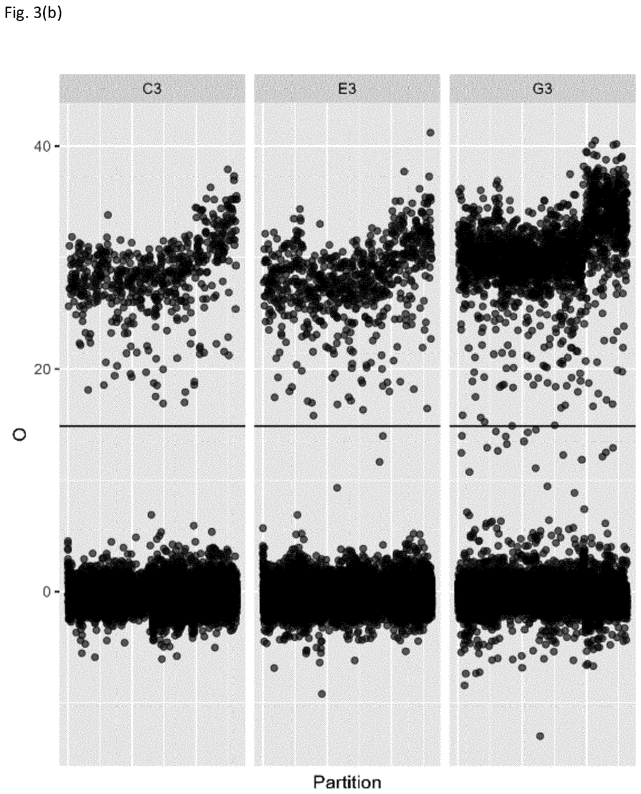

Fig. 3 shows the effects of the inventive method in channel O. In Fig. 3(a), the data in channel O show the various errors caused by cross-talk, competition effects and optical artifacts. The cross-talk can be seen in the additional groups of partitions, especially in well G3. Optical artifacts are clearly visible in well C3 due to jumps in the fluorescence intensities within the bands. The fluorescence intensities of the same wells in channel O are shown In Fig. 3(b), after application of the inventive method. The horizontal line represents the limit value.

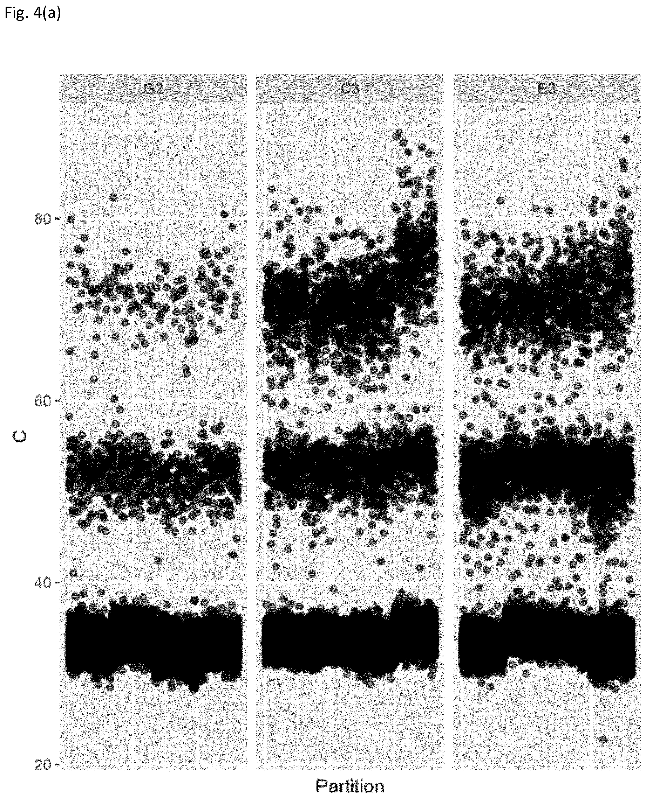

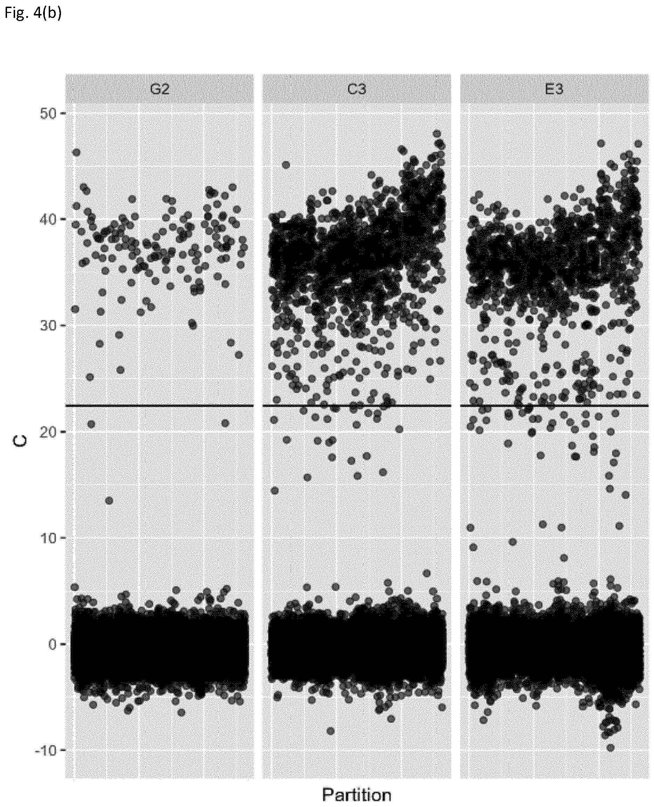

Fig. 4 shows the effect of cross-talk correction in channel C. In Fig. 4(a), the effect of cross-talk can be seen in the additional group of partitions. Fig. 4(b) shows the same data after processing by theinventive method. The horizontal line again represents the limit value.

The claimed method is thus suitable for identification and correction of errors in multichannel raw fluorescence data obtained by digital polymerase chain reaction, dPCR, in particular by processing of the raw-data output of the dPCR system to correct for background noise and artifacts, to correct for cross-talk between the channels, and to correct for competitive effects between the parallel reactions.

While various embodiments of the present disclosure have been described above, it should be understood that they have been presented by way of example only, and not by way of limitation. Likewise, the various diagrams may depict an example architectural or configuration, which are provided to enable persons of ordinary skill in the art to understand exemplary features and functions of the present disclosure. Such persons would understand, however, that the present disclosure is not restricted to the illustrated example architectures or configurations, but can be implemented using a variety of alternative architectures and configurations. Additionally, as would be understood by persons of ordinary skill in the art, one or more features of one embodiment can be combined with one or more features of another embodiment described herein. Thus, the breadth and scope of the present disclosure should not be limited by any of the above-described exemplary embodiments.

It is also understood that any reference to an element herein using a designation such as "first," "second," and so forth does not generally limit the quantity or order of those elements. Rather, these designations can be used herein as a convenient means of distinguishing between two or more elements or instances of an element. Thus, a reference to first and second elements does not mean that only two elements can be employed, or that the first element must precede the second element in some manner.

Additionally, a person having ordinary skill in the art would understand that information and signals can be represented using any of a variety of different technologies and techniques. For example, data, instructions, commands, information, signals, bits and symbols, for example, which may be referenced in the above description can be represented by voltages, currents, electromagnetic waves, magnetic fields or particles, optical fields or particles, or any combination thereof.

A skilled person would further appreciate that any of the various illustrative logical blocks, units, processors, means, circuits, methods and functions described in connection with the aspects disclosed herein can be implemented by electronic hardware (e.g., a digital implementation, an analogue implementation, or a combination of the two), firmware, various forms of program or design code incorporating instructions (which can be referred to herein, for convenience, as "software" or a "software unit"), or any combination of these techniques.

To clearly illustrate this interchangeability of hardware, firmware and software, various illustrative components, blocks, units, circuits, and steps have been described above generally in terms of their functionality. Whether such functionality is implemented as hardware, firmware or software, or a combination of these techniques, depends upon the particular application and design constraints imposed on the overall system. Skilled artisans can implement the described functionality in various ways for each particular application, but such implementation decisions do not cause a departure from the scope of the present disclosure. In accordance with various embodiments, a processor, device, component, circuit, structure, machine, unit, etc. can be configured to perform one or more of the functions described herein. The term "configured to" or "configured for" as used herein with respect to a specified operation or function refers to a processor, device, component, circuit, structure, machine, unit, etc. that is physically constructed, programmed and/or arranged to perform the specified operation or function.

Furthermore, a skilled person would understand that various illustrative methods, logical blocks, units, devices, components and circuits described herein can be implemented within or performed by an integrated circuit (IC) that can include a general purpose processor, a digital signal processor (DSP), an application specific integrated circuit (ASIC), a field programmable gate array (FPGA) or other programmable logic device, or any combination thereof. The logical blocks, units, and circuits can further include antennas and/or transceivers to communicate with various components within the network or within the device. A general purpose processor can be a microprocessor, but in the alternative, the processor can be any conventional processor, controller, or state machine. A processor can also be implemented as a combination of computing devices, e.g., a combination of a DSP and a microprocessor, a plurality of microprocessors, one or more microprocessors in conjunction with a DSP core, or any other suitable configuration to perform the functions described herein. If implemented in software, the functions can be stored as one or more instructions or code on a computer-readable medium. Thus, the steps of a method or algorithm disclosed herein can be implemented as software stored on a computer-readable medium.

Computer-readable media includes both computer storage media and communication media including any medium that can be enabled to transfer a computer program or code from one place to another. A storage media can be any available media that can be accessed by a computer. By way of example, and not limitation, such computer-readable media can include RAM, ROM, EEPROM, CD-ROM or other optical disk storage, magnetic disk storage or other magnetic storage devices, or any other medium that can be used to store desired program code in the form of instructions or data structures and that can be accessed by a computer.

Additionally, memory or other storage, as well as communication components, may be employed in embodiments of the present disclosure. It will be appreciated that, for clarity purposes, the above description has described embodiments of the present disclosure with reference to different functional units and processors. However, it will be apparent that any suitable distribution of functionality between different functional units, processing logic elements or domains may be used without detracting from the present disclosure. For example, functionality illustrated to be performed by separate processing logic elements, or controllers, may be performed by the same processing logic element, or controller. Hence, references to specific functional units are only references to a suitable means for providing the described functionality, rather than indicative of a strict logical or physical structure or organization.

Various modifications to the implementations described in this disclosure will be readily apparent to those skilled in the art, and the general principles defined herein can be applied to other implementations without departing from the scope of this disclosure. Thus, the disclosure is not intended to be limited to the implementations shown herein, but is to be accorded the widest scope consistent with the novel features and principles disclosed herein, as recited in the claims below.

## Claims

1. A method for error correction in multichannel raw fluorescence data obtained by digital polymerase chain reaction, dPCR, the method comprising the steps of:
(a) identifying partitions providing aberrant fluorescence readings in a first fluorescence channel,
(b) comparing the fluorescence readings of at least some of the partitions providing aberrant fluorescence reading in the first fluorescence channel, with the fluorescence reading of the same partitions in a second fluorescence channel,
(c) adjusting the fluorescence readings of the partitions providing aberrant fluorescence readings in the first fluorescence channel based on the result of the comparison in (b).

2. The method according to claim 1, the method further comprising identifying partitions providing positive and negative fluorescence readings in the first fluorescence channel, wherein partitions providing aberrant fluorescence readings are distinguished from the partitions providing positive and negative fluorescence readings by determining local minimums in a histogram of fluorescence intensities of the partitions registered in the first fluorescence channel.

3. The method according to claim 1 or 2, wherein in step (b) fluorescence intensity in the partitions providing aberrant fluorescence reading in the first fluorescence channel is compared with fluorescence intensity in the same partitions in the second fluorescence channel, for determining an error type of the aberrant fluorescent reading.

4. The method according to claim 3, wherein a crosstalk error is determined as type of error in step (b), if a substantial number of the partitions providing aberrant fluorescence reading in the first fluorescence channel provides a positive fluorescence reading in the second fluorescence channel.

5. The method according to claim 3 or 4, wherein a crosstalk error is determined as type of error in step (b), if there are no partitions or essentially no partitions that provide both a positive fluorescence reading in the second fluorescence channel and negative fluorescence reading in the first fluorescence channel.

6. The method according to any of claims 3 to 5, wherein a competitive reaction error is determined as type of error in step (b), if a substantial number of the partitions providing aberrant fluorescence reading in the first fluorescence channel gives aberrant fluorescence intensity in both the first channel and at least the second channel, and/or a substantial number of the partitions providing aberrant fluorescence reading in the first fluorescence channel gives a positive fluorescent reading in at least the second channel provided also that there is a substantial number of partitions providing both a positive fluorescence reading in the second fluorescence channel and negative fluorescence reading in the first fluorescence channel.

7. The method according to any one of the preceding claims, wherein a linear regression method is used for compensating the error, by estimating the strength of the error based on fluorescence intensity data from partitions which are affected by the errors and from partitions which are unaffected by the errors.

8. The method according to any one of the preceding claims, wherein in step (b) fluorescence intensity in the partition providing aberrant fluorescence reading in the first fluorescence channel is compared with fluorescence intensity of the same partition in more than one second fluorescence channel, preferably with fluorescence intensities of the same partition in all of the available fluorescence channels.

9. The method according to any one of the preceding claims, wherein steps (a) to (c) are repeated for at least one other fluorescence channel, preferably for all of the available fluorescence channels.

10. A machine-readable medium storing instructions that, when executed by a computer, cause it to perform the method according to any one of claims 1 to 9.

11. A digital polymerase chain reaction, dPCR, device configured to carry out the method of any one of claims 1 to 9.
